# EUROPEAN PATENT APPLICATION

(11) **EP 1 026 482 A1**
(43) Date of publication of application: **09.08.2000**
(21) Application number: 99123209.1
(22) Date of filing: 25.11.1999
(51) Int. Cl.: G01F 23/296, B05B 17/06

(54) **Ultrasonic nebuliser**

(30) Priority: 04.02.1999 SE 9900369
(71) Applicant: Siemens-Elema AB, 171 95 Solna 1 (SE)
(72) Inventor: Cewers, Göran, 223 50 Lund (SE)

(57) **Abstract**

An ultrasonic nebuliser (1) includes a nebulisation chamber (4) for holding a liquid (5) to be nebulised, the liquid (5) being delimited by an upper boundary (6) within the chamber (4); and a nebulisation source (2,10) acoustically couplable to the liquid (5) within the chamber (4) to provide therein an ultrasonic output at an amplitude to cause nebulisation **characterised in that** the nebulisation source (2,10) is controllable to vary the amplitude of the output to provide a measurement period during which no nebulisation occurs and in that there is provided a sonar device (2,11) adapted to measure during the measurement period a time interval between an emission of an acoustic pulse (13) towards the boundary (6) and a detection of a component of the emitted acoustic pulse reflected from the boundary (6) and to provide an output signal dependent on the measured time interval for use in determining location information on the boundary (6) within the chamber (4).

## Description

The present invention relates to an ultrasonic nebuliser and in particular to one having an output controllable in dependence of the level of liquid available for nebulisation.

Ultrasonic nebulisers are devices which utilise a source of ultrasound, such as for example a piezoelectric crystal oscillator, acoustically coupled to a liquid in a nebulising chamber in order to generate an aerosol of small liquid droplets in a space above the liquid boundary. The generated aerosol may be used for any desired purpose such as humidification or medication. Such nebulisers are often found as a component in a breathing circuit of a mechanical ventilator, where they are employed in the delivery of controlled doses of anaesthetic or other additive into a breathing gas for supply to a patient.

It is important, particularly in the medical field, to be able to monitor the level of liquid in the nebulising chamber. This may be for example, in order to maintain a supply of liquid throughout mechanical ventilation or to monitor the dosage of liquid delivered into the breathing gas.

One known ultrasonic nebuliser which is provided with a liquid level indicator is disclosed in US 3,839,651. This nebuliser uses a temperature sensitive resistance element which is thermally coupled to the liquid within the nebulising chamber. The current in an electrical circuit containing this element is dependent on the amount of liquid within the chamber and is used to remove power from the oscillator and to provide a visible indication when the liquid level falls to a predetermined minimum. One problem with such a level indicator is that it is relatively insensitive to small changes in liquid level which are likely to occur between successive, or closely spaced inspiration periods of a patient breathing cycle.

It is an aim of the present invention to provide an ultrasonic nebuliser having a level indicator capable of sensing such small changes.

This is achieved by the nebuliser according to and characterised by claim 1. By controlling the amplitude of the nebulisation source to provide periods where no nebulisation occurs, possibly by providing periods of zero amplitude output, a sonar device which employs echo ranging techniques may be used to measure the location of the upper boundary of the liquid within the nebulisation chamber. This provides a relatively sensitive means for identifying changes in the location of the liquid boundary from which, for example, the amount of liquid within the nebulisation chamber may be calculated.

Preferably, one piezoelectric crystal is employed as both the nebulisation source and as the sonar device. This allows existing nebulising chambers and sources to be used with only modifications to the electronic circuitry used to control the crystal being necessary. Moreover, by using only the one crystal a major component cost saving is achieved as compared with employing separate sonar and nebulisation sources.

Usefully, a difference forming means is used to enable differences in the location of the liquid boundary to be determined. The determined difference may for example be used to monitor the amount of liquid nebulised between measurement periods or to monitor the effect of different known crystal driving currents on the liquid boundary during a single measurement period. Both of these monitoring modes may then be employed to calibrate the nebulisation source and to control the amplitude or duration of the ultrasonic output from the source in order to, for example, more reliably provide a required amount of nebulisation or to remove power from the source if a minimum liquid level is reached.

Particularly useful is the latter mode of monitoring since a calibration of the output of the nebulisation source may be made before generating any nebulised liquid.

These and other advantages will be better understood from a consideration of the following description of an embodiment and operating modes of the present invention, made with reference to the drawings of the accompanying figures of which:

Figure 1 shows a schematic representation of an embodiment of a nebuliser and illustrates one mode of operation according to the present invention.

Figure 2 shows a schematic representation of a nebuliser illustrating a further mode of operation according to the present invention.

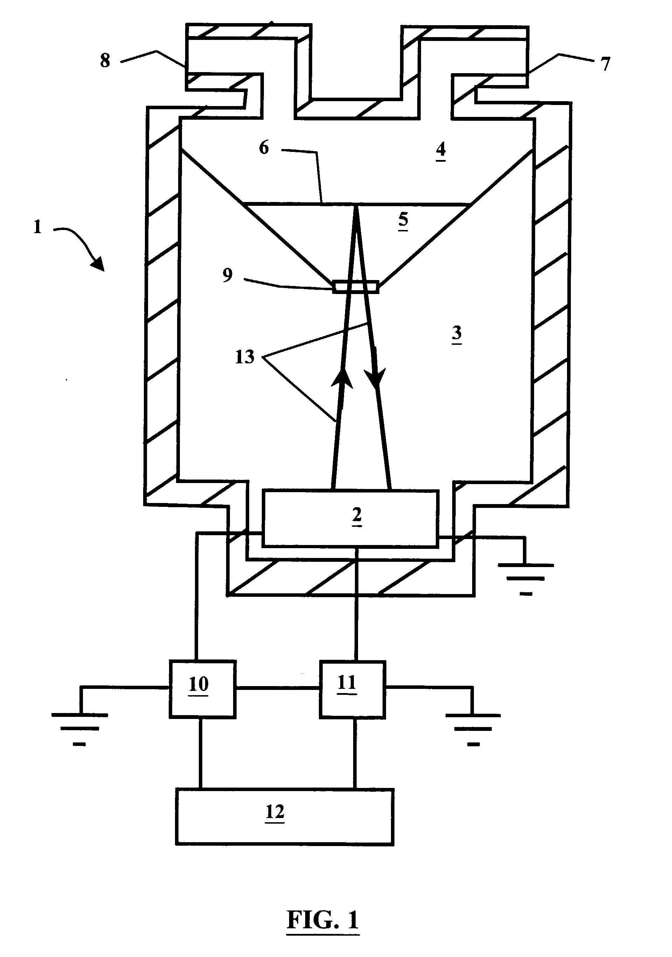

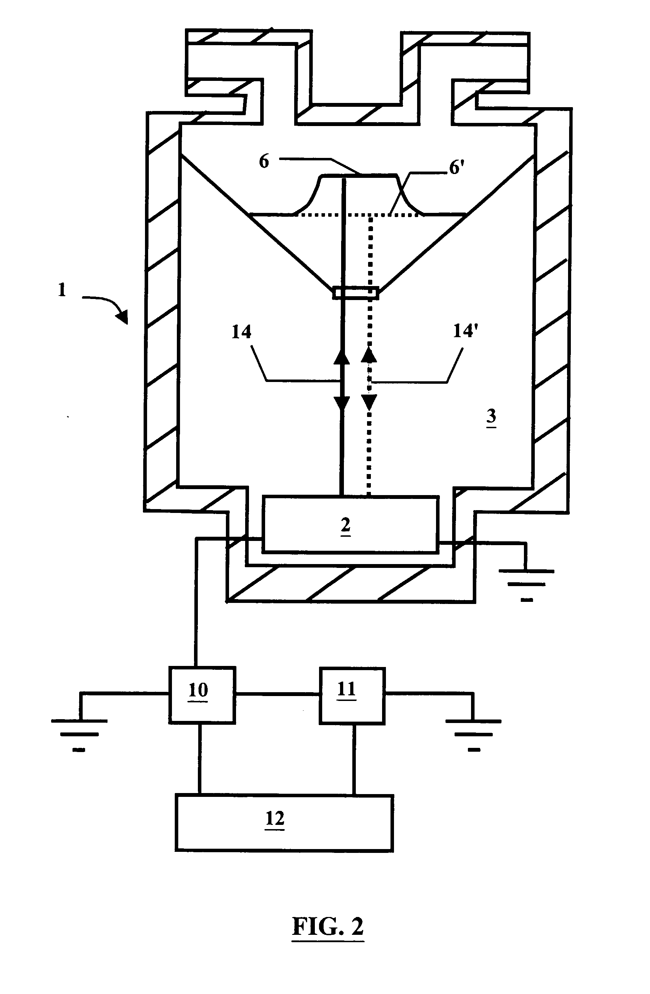

Considering now Figures 1 and 2 in which an ultrasonic nebuliser is shown generally at 1. The nebuliser 1 of Figures 1 and 2 have the same basic components but different modes of operation, which modes will be described separately for each of the Figures 1 and 2.

The ultrasonic nebuliser 1 of Figures 1 and 2 comprises a ultrasonic oscillator 2, here in the form of a piezoelectric transducer, which is located in a water chamber 3 above which is a nebulisation chamber 4. A liquid 5 for nebulisation is held within the nebulisation chamber 4 so that in use a space into which nebulised liquid passes remains in the chamber 4 above an upper boundary 6 of the liquid 5. Gas may be introduced into the nebulisation chamber 4 to flow from an inlet 7 to an outlet 8 through the space above the upper boundary 6 and remove from the chamber 4 liquid droplets formed during nebulisation. A thin membrane 9 separates the water chamber 3 from the nebulisation chamber 4 so that ultrasonic energy from the oscillator 2 can pass readily there through with the result that the oscillator 2 "sees" essentially only a single body of liquid, terminating at the upper boundary 6. Oscillator driver means 10 is connected in an electrical circuit with the oscillator 2 and is arranged to drive the oscillator 2 to generate a controllable, variable amplitude ultrasonic signal for output into the water chamber 3. Sonar detection means 11 is also provided in electrical connection with the oscillator drive means 10 and with the oscillator 2. The detection means 11 includes conventional timer circuitry (not shown) which is arranged to measure the time taken for an ultrasonic sonar pulse to travel from the oscillator 2 to the upper liquid boundary 6 and back again. The detection means 11 is also adapted to output a signal representative of this measured transit time.

The driver means 10 and the sonar detection means 11 are readily realisable by those skilled in the art using conventional electrical engineering methodology and an understanding of the principles of their function, as provided herein.

A controller unit 12, for example in the form of a suitably programmed computer, is operably connected to both the driver means 10 and to the sonar detection means 11 and is adapted to provide control of the driver means 10 and to calculate the location of the upper liquid boundary 6 within the nebulisation chamber 4 from the output signal of the sonar detection means 11.

Considering now the nebuliser 1 of Figure 1 and its mode of operation, as illustrated by the arrows 13 which show the path of the sonar pulse. In use the controller unit 12 provides control instructions to the driver means 10 for generating a periodic variation in the amplitude of the ultrasonic energy output by the oscillator 2. The variation is such that nebulisation periods, during which is output high amplitude ultrasound sufficient to cause nebulisation, alternate with measurement periods, during which is output only ultrasound sonar pulses at an amplitude, for example zero amplitude, insufficient to effect i.e. disturb the location of the upper boundary 6 to a measurable extent.

During a measurement period a trigger signal is sent to the sonar detection means 11 corresponding to the oscillator 2 being driven to generate an ultrasonic sonar pulse. The trigger signal, which may conveniently be provided either by the controller unit 12 or the driver means 10, initiates the start of timing by the timer circuitry. The timing is stopped when the receipt of a reflected component of the generated ultrasonic pulse is detected at the oscillator 2. This detection is facilitated by the use of a piezoelectric transducer as the oscillator 2. It is well known that ultrasonic energy incident on such a piezoelectric transducer 2 can cause detectable changes in the electrical properties of an electrical circuit in which the transducer 2 is included. Thus in the present embodiment the receipt of the reflected pulse is detected using such known circuitry within the detection means 11. The pulse emission and detection is optionally carried out a plurality of times throughout the measurement period and an average location determination is made within the controller unit 12 based on the averaged value of the measured transit times. The determined location may be used within the controller unit 12 to provide a control signal which inhibits the operation of the driver means 10 when a location corresponding to a minimum level is detected.

Optionally, the controller unit 12 may be programmed to compare the presently determined location with a previously determined location, having a known temporal relationship to the presently determined location and preferably one made in a consecutive measurement period, so that the amount of liquid nebulised during an intervening nebulisation period or periods may be calculated within the controller unit 12 to provide dose information. This dose information may then be used by the controller unit 12 in the control of the driver means 10 to regulate one or both of the amplitude of the ultrasound generated for nebulisation and the duration of the nebulisation period in order to obtain a desired dose from the nebuliser 1.

Considering now the nebuliser 1 of Figure 2 and its mode of operation, as illustrated by the solid 14 and broken 14' arrows showing paths of the sonar pulses. In use the controller unit 12 controls the driver means 10 to provide the piezoelectric transducer 2 with a known, variable amplitude driving force to generate a corresponding variable amplitude ultrasonic output into the water chamber 3. The driving means 10 is controlled to provide from the oscillator 2 at least one nebulisation period, during which is output high amplitude ultrasound sufficient to cause nebulisation, and at least one measurement period, during which is output ultrasound at amplitudes less than is necessary to cause nebulisation.

During each measurement period the controller unit 12 controls the driver unit 10 to generate at least two different amplitudes of ultrasound to provide distinguishable and different locations of at least part of the upper boundary 6,6'. As illustrated in Figure 2 one output amplitude is chosen so as not to effect (i.e. disturb) the location of the upper boundary 6' to a measurable extent, for example a zero amplitude output, and the other output amplitude is chosen to provide a localised change in the upper boundary 6. The sonar detection means 11 and the driver means 10 operate co-operatively to provide two measurements of transit times; one for a sonar pulse travelling along the path 14 which measures the location of the boundary 6 and the other for a sonar pulse travelling along the path 14' which measures the location of the boundary 6'. These measurements are carried out in a manner analogous to that described in respect of the transit time measurements of Figure 1.

The controller unit 12 is operably connected to the sonar detection means 11 to receive outputs from the means 11 representative of the two measured transit times. A difference calculation between the locations of the boundaries 6,6' is made within the controller unit 12. The controller unit 12 is further adapted to calculate the rate of change of location of the boundary with supplied driving force using the calculated difference and from the knowledge of the amplitude of the driving force supplied by the driving means 10 to generate the two upper boundary locations 6,6'. From this rate of change the controller unit 12 generates an estimate of the driving force required to be provided to the oscillator 2 in order to generate a desired degree of nebulisation within the nebuliser 1 and controls the driver means 10 accordingly.

## Claims

1. An ultrasonic nebuliser (1) including a nebulisation chamber (4) for holding a liquid (5) to be nebulised, the liquid (5) being delimited by an upper boundary (6,6') within the chamber (4); and an ultrasonic nebulisation source (2,10) acoustically couplable to the liquid (5) to provide therein nebulisation **characterised in that** the nebulisation source (2,10) is controllable to generate a variable amplitude ultrasound output to provide a measurement period during which no nebulisation occurs and in that there is provided a sonar device (2,11) adapted to measure during the measurement period a time interval between an emission of an acoustic pulse (13,14,14') towards the upper boundary (6,6') and a detection of a component of the emitted acoustic pulse reflected from the boundary (6,6') and to provide an output signal dependent on the measured time interval for use in determining location information on the upper boundary (6,6') within the chamber (4).

2. A nebuliser as claimed in Claim 1 **characterised in that** the sonar device (2,11) is arranged to emit the acoustic pulse through the liquid (5) to the upper boundary (6,6').

3. A nebuliser as claimed in Claim 2 **characterised in that** there is provided a piezoelectric tranducer (2) adapted to generate the controllable ultrasonic output of the nebulisation source (2,10) and to emit and receive the acoustic pulse (13,14,14') of the sonar device (2,11) during the measurement period.

4. A nebuliser as claimed in any preceding claim **characterised in that** there is further provided a difference forming means (12) adapted to receive the output signal from the sonar device (2,11) and to determine changes in the location of upper boundary (6,6') within the chamber (4) derived from a difference between the received signal and a reference signal.

5. A nebuliser as claimed in Claim 4 **characterised in that** the nebulisation source (2,10) is controllable to provide during the measurement period a first output amplitude selected to produce a first location of the upper boundary (6 or 6') and a second output amplitude selected to produce a second, different, location of the boundary (6' or 6); in that the sonar device (2,11) is adapted provide a first output signal associated with the first amplitude and a second output signal associated with the second amplitude; and in that the difference forming means (12) is adapted to employ one signal as the received signal and the other as the reference signal.

6. A nebuliser as claimed in claim 5 **characterised in that** nebulisation source (2,10) is controllable to provide one or other of the first and second amplitudes at a level selected to cause substantially no disturbance of the location of the upper boundary (6).

7. A device as claimed in any of the claims 2 to 4 **characterised in that** the nebulisation source (2,10) is controllable to provide during the measurement period only an output amplitude selected to cause substantially no disturbance to the upper boundary (6).

8. A device as claimed in claim 7 **characterised in that** the difference forming means (12) includes a memory means for storing received signals from a plurality of measurement periods and in that the difference forming means is adapted (12) to employ a previously stored signal as the reference signal.

9. A device as claimed in claim 8 in that the difference forming means (12) is adapted to derive the difference between signals received during consecutive measurement periods.

10. A device as claimed in any of the claims 4 to 9 **characterised in that** the difference forming means (12) is adapted to provide an output signal dependent on the derived difference for use in controlling one or both of the amplitude and the duration of the output of the nebulisation source (2,10).
